(19) **Europäisches Patentamt**

**European Patent Office**

**Office européen des brevets**

(11) Numéro de publication : **0 426 568 B1**

(12) # FASCICULE DE BREVET EUROPEEN

(45) Date de publication du fascicule du brevet :
**17.06.92 Bulletin 92/25**

(51) Int. Cl.$^5$ : **A61B 6/00**

(21) Numéro de dépôt : **90403087.1**

(22) Date de dépôt : **31.10.90**

(54) **Equipement gamma caméra à deux têtes détectrices.**

(30) Priorité : **02.11.89 FR 8914356**

(43) Date de publication de la demande :
**08.05.91 Bulletin 91/19**

(45) Mention de la délivrance du brevet :
**17.06.92 Bulletin 92/25**

(84) Etats contractants désignés :
**BE DE GB LU NL**

(56) Documents cités :
**EP-A- 0 049 562**
**EP-A- 0 251 487**
**EP-A- 0 320 741**
**DE-A- 2 323 710**
**US-A- 4 645 933**

(73) Titulaire : **SOPHA MEDICAL**
**9, place de la Madeleine**
**F-75008 Paris (FR)**

(72) Inventeur : **Pierfitte, Michel**
**Cabinet Ballot-Schmit, 7, rue Le Sueur**
**F-75116 Paris (FR)**
Inventeur : **Pare, Christian**
**Cabinet Ballot-Schmit, 7, rue Le Sueur**
**F-75116 Paris (FR)**
Inventeur : **De La Barre, François**
**Cabinet Ballot-Schmit, 7, rue Le Sueur**
**F-75116 Paris (FR)**

(74) Mandataire : **Schmit, Christian Norbert Marie**
**et al**
**Cabinet Ballot-Schmit 7, rue Le Sueur**
**F-75116 Paris (FR)**

## Description

L'invention se rapporte au domaine des gamma cameras, ou cameras à scintigraphie et plus particulièrement à un équipement gamma caméra à deux têtes détectrices.

Classiquement (voir US-A- 4 645 933), les équipements d'examen médical par scintigraphie sont adaptés soit à une analyse dite "tomographique", soit à une analyse dite "corps entier" Pour une analyse dite "tomographique", une tête détectrice qui comporte un collimateur de rayonnement gamma, un scintillateur et un ensemble détecteur associé, est déplacée en rotation autour du patient de façon à former des images selon différents plans d'un organe à analyser.

Pour une analyse dite "corps entier", deux têtes de caméra respectivement placées au-dessus et au-dessous du lit ad hoc portant le patient, permettent de former à partir des rayonnements reçus par les deux têtes des images de plans parallèles aux faces d'entrée des têtes. Pour un examen de ce type, classiquement, les deux têtes sont déplacées simultanément, la tête située au-dessus du lit du patient étant déplacée parallèlement au lit en même temps que le statif dans son ensemble tandis que la tête située au-dessous du lit du patient est déplacée sur des rails maintenus au sol par une jambe de force s'étendant sur toute la longueur des rails. Une telle installation est lourde, encombrante et pour autant ne permet pas une utilisation souple du matériel. En particulier, les têtes détectrices sont spécialisées : une seule tête montée pour autoriser une large gamme de déplacements rotatifs pour l'analyse tomographique, ou deux têtes couplées pour un déplacement longitudinal pour l'analyse corps entier.

L'invention a pour objet un équipement gamma caméra, de structure relativement plus simple que les équipements antérieurs et qui, de plus, permet de réaliser soit des analyses tomographiques, soit des analyses corps entier, au choix de l'opérateur, avec seulement deux têtes détectrices.

Selon l'invention, l'équipement gamma caméra comporte un statif sur lequel est montée une première tête détectrice, une seconde tête détectrice basse et est caractérisé en ce que, pour permettre des examens de deux types, type corps entier utilisant les deux têtes détectrices et type tomographique n'utilisant que la première tête détectrice, l'équipement comporte des rails de roulement sur lesquels la seconde tête détectrice peut être déplacée entre une position active où son support est verrouillé au statif, et une position d'attente où son support est verrouillé aux rails de roulement, en bout de rails, cette seconde tête étant, quelle que soit sa position toujours alimentée en même temps que la première.

Comme indiqué ci-dessus, cet équipement comporte un tête détectrice placée en permanence au-dessus du lit ad hoc et susceptible de tourner pour le mode d'analyse tomographique, et une deuxième tête de caméra, déplaçable sur des rails fixés au sol pour être soit mise en attente en bout de rails lorsque l'équipement fonctionne selon le mode tomographique, soit amenée sous le lit ad hoc par roulement sur les rails pour le mode d'analyse corps entier.

Un problème de ce type d'équipements est lié au fait qu'une tête de gamma caméra est un ensemble lourd, de l'ordre de 400 kgs et que la seconde tête, lorsqu'elle est mise en attente en bout de rails doit être alimentée en permanence et, étant en porte à faux, doit être associée à un ensemble mécanique suffisamment solide pour éviter toute rupture, notamment par arrachement.

Ce problème pourrait être résolu comme pour les équipements d'analyse corps entier à deux têtes selon l'art antérieur, en prévoyant, sur toute la longueur des rails sur lesquels la deuxième tête est déplacée, une jambe de force. Mais cela augmente l'emprise au sol. Ou bien on réalise des ancrages solides, mais cela nécessite que le sol soit robuste et compact : Ceci est rarement le cas. Un autre objet de l'invention est donc de réaliser un équipement gamma caméra à deux têtes détectrices dans lequel l'ensemble mécanique associé à la deuxième tête, pour éviter un arrachement lorsque cette deuxième tête est mis en attente, est plus simple tout en étant satisfaisant ; cet équipement peut, en outre, être installable sur n'importe quel type de sol et même éventuellement, sur un dalle flottante.

Selon un autre objet de l'invention, l'équipement est tel que les rails de roulement sur lesquels la seconde tête détectrice est déplaçable sont solidaires d'une platine de liaison sur laquelle s'exerce également le poids de statif, le statif portant la première tête détectrice formant contrepoids à la seconde tête détectrice.

L'invention sera mieux comprise et d'autres caractéristiques apparaitront à l'aide de la description qui suit en référence aux figures annexées.

La figure 1 est un schéma d'ensemble de l'équipement gamma caméra selon l'invention ;

La figure 2 est le schéma de l'emprise au sol des différents éléments formant l'équipement gamma caméra selon l'invention ;

La figure 3 est un schéma plus détaillé de l'ensemble mécanique associé à la deuxième tête détectrice.

La figure 4 illustre le système de visualisation des positions de la seconde tête détectrice.

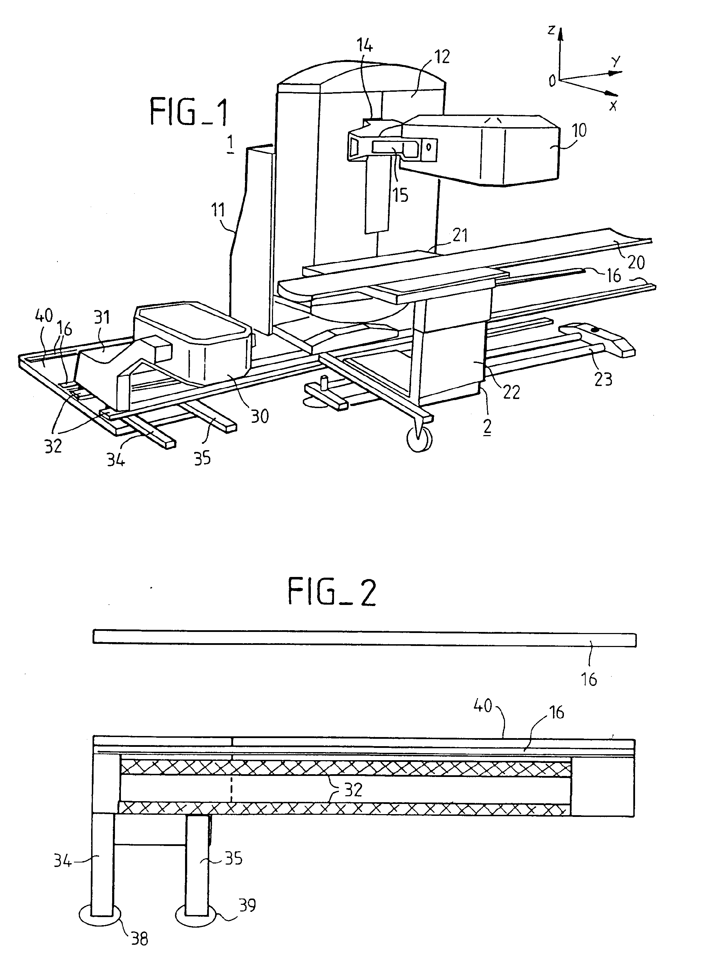

L'équipement à gamma caméra selon l'invention tel que représenté sur la figure 1 comporte un ensemble d'éléments classiques définis dans un repère (OX,Y,Z):

- un statif 1, c'est-à-dire un socle massif, lourd, et stable qui comporte une partie arrière 11, un support 12 susceptible de tourner dans un plan vertical, (YOZ), dans lequel est fixé un bras 14 terminé par un étrier 15. Cet étrier 15 supporte une première tête détectrice 10 pivotante autour d'un axe (parallèle à OY) défini par les points de fixation de la tête détectrice 10 à l'étrier 15. Ce statif est éventuellement déplaçable avec la tête qu'il supporte, sur des rails 16 selon OY.

- une table d'examen 2 comporte un lit 20 monté en porte à faux sur un support 21, lui-même en porte à faux sur un pied 22 de façon qu'une seconde tête détectrice puisse être placée sous le lit ; ce lit est réalisé en matériau transparent au rayonnement gamma utilisé pour l'examen. Le pied du lit 22 est monté coulissant sur des rails 23 fixés au sol, parallèles à l'axe OY.

- une seconde tête détectrice 30 est montée en porte à faux sur un support 31, l'ensemble, d'une masse de l'ordre de 400 kgs étant déplaçable au ras du sol sur des rails 32.

Préférentiellement, ces rails 32 sont solidaires du statif 1 de façon que le statif fasse contrepoids pour la seconde tête détectrice. Lorsque celle-ci est en position d'attente ou "PARK" à l'extrémité des rails 32, et du fait qu'elle est montée en porte à faux, des jambes de force 34, 35 sont prévues seulement à l'extrémité des rails 32, pour éviter un arrachement. Ces jambes de force sont soudées, pour ne pas jouer, à la platine de base 40 portant également le chemin de roulement de la roue avant du statif 1, et les rails 32 de la seconde tête. Cet ensemble compact est installable sur n'importe quel type de sol et ne nécessite pas de fixation particulièrement rigide, le statif portant la première tête faisant alors contrepoids au porte à faux de la deuxième tête détectrice.

En position d'attente, la deuxième tête détectrice, 30, toujours alimentée en même temps que la première, est en plus verrouillée sur les rails 32. En position active, la seconde tête est déverrouillée des rails 32 en même temps qu'elle est verrouillée au statif 1.

La figure 2 représente schématiquement l'emprise au sol de l'équipement gamma caméra multi-analyses selon l'invention, sans sa partie correspondant à la table d'examen 2. Les mêmes références que sur la figure 1 désignent les mêmes éléments, à savoir 16 pour les rails de guidage de l'ensemble statif avec sa première tête détectrice, 32 pour les rails à billes de guidage de l'ensemble support 31 et deuxième tête détectrice, 34 et 35 pour les jambes de force disposées en bout des rails 16 ; ces différents éléments sont portés par la platine de base 40.

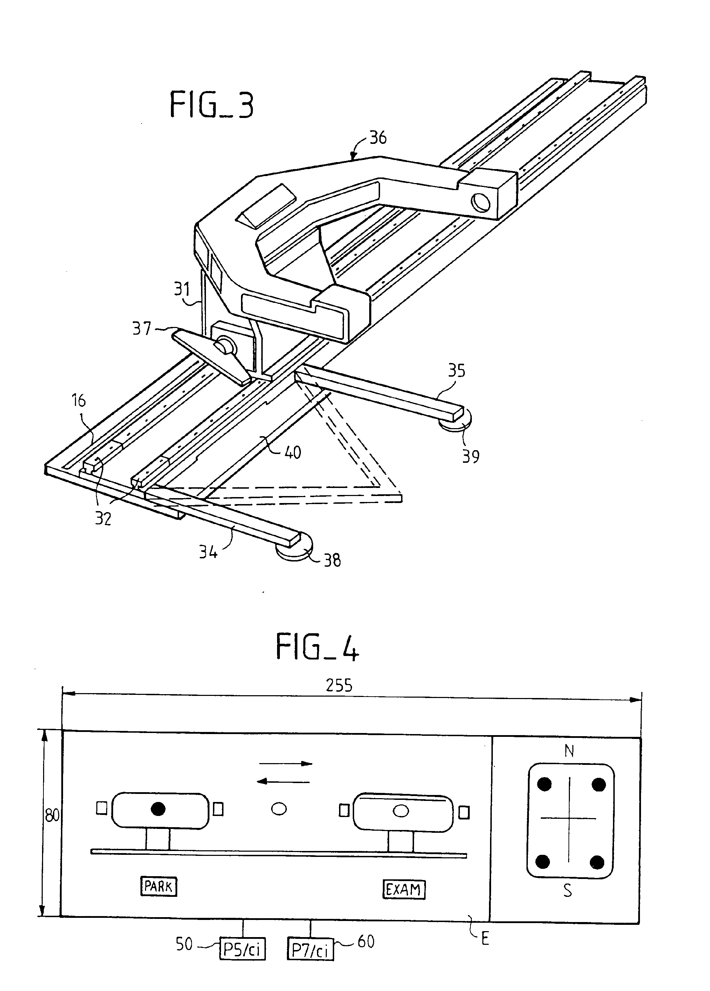

La figure 3 illustre de façon plus détaillée l'ensemble mécanique associé à la seconde tête détectrice, elle-même non représentée sur cette figure pour permettre de montrer les différents éléments. On distingue notamment un étrier 36 destiné à porter la seconde tête détectrice 30, sur un support 31 qui contient l'alimentation du détecteur, une pédale 37 de verrouillage et déverrouillage de ce support sur les rails 32, les jambes de force 34 et 35 soudées à la platine de base 40 commune aux rails 32 et au chemin de roulement 16 de la roue avant du statif. L'ensemble statif portant la première tête détectrice a une masse de l'ordre de 900 Kgs et le support 31 avec la seconde tête détectrice une masse de l'ordre de 400 Kgs.

L'ensemble est donc équilibré par le fait
- que la platine de base est fixe au sol
- que le poids du statif s'exerce pour moitié sur cette platine de base 40 par l'intermédiaire de la roue avant appuyant sur le chemin de roulement correspondant aménagé également sur la platine de base,
- et que les jambes de force qui lui sont soudées exercent une réaction sur le sol qui permet d'empêcher la seconde tête de basculer en arrachant les rails, à l'extrémité où la seconde tête est mise en attente, et lorsque le statif est à l'autre extrémité des rails.

Bien entendu, dans le mode de réalisation représenté une platine de base unique a été utilisée. Il est tout à fait possible d'utiliser deux platines différentes pour le roulement du statif et les rails portant la deuxième tête détectrice, mais dans ce cas les deux platines doivent être liées par un élément de liaison fixe. Là encore la réaction de basculement doit être exercée via le verrouillage de la deuxième tête sur le statif.

Pour permettre un équilibrage correct de l'ensemble, les jambes de force 34 et 35 sont prévues avec des vérins de réglage, respectivement 38 et 39 qui permettent d'ajuster la hauteur des jambes de force par rapport au sol. Ces jambes de force ont été représentées orthogonales à la direction des rails 32 sur les figures 1 à 3. En pratique, le système de jambes de force permettant d'éviter l'arrachement en bout de rails peut être également formé de deux jambes de force obliques réunies en un point commun où est disposé un vérin de réglage de la hauteur par rapport au sol, selon la disposition représentée en pointillés sur la figure 3.

Cette organisation a évidemment pour avantage de réduire l'encombrement au sol. Dans les deux cas, jambes de force orthogonales et jambes de force obliques, les zones de soudure de ces jambes à la platine de base sont suffisamment écartées pour encadrer le support 31 sur les rails 32, dans la position d'attente.La platine de base 40, peut être une platine antisysmique si nécessaire pour éviter de transmettre les vibrations dans les régions souvent soumises à des tremblement de terre.

Le verrouillage du support 31 portant la deuxième tête détectrice a été représenté comme une pédale qui actionne un système de blocage. Le système de blocage comporte un organe mécanique s'engageant dans le statif et dans la deuxième tête (en examen corps entier) ou dans le statif et dans les rails 32 ou la platine 40 (en utilisation tomographique). Cet organe est suffisamment long pour s'engager temporairement dans les trois en même temps lorsqu'on passe d'une utilisation à un autre. Un ressort de maintien peut être utilisé pour le blocage dans un sens ou dans l'autre. Un système de sécurité électrique peut lui être adjoint de façon que l'équipement reconnaisse un bon verrouillage, aussi bien en position active sur le statif, qu'en position d'attente sur les rails 32, un système de visualisation de fin de course peut également être prévu. Un mode de réalisation d'un tel système de visualisation est représenté sur la figure 4 : un écran de visualisation E comporte un certain nombre de symboles préaffichés en permanence et notamment la position active, marquée "EXAM" de la tête détectrice basse, la position d'attente marquée "PARK" de la tête détectrice basse, et une figuration du chemin de déplacement de cette tête. Des voyants sont prévus pour indiquer la position réelle de la tête basse dans l'équipement. Les voyants au centre des deux positions sont rouge ou vert selon la position de la tête, la couleur verte marquant le verrouillage de la tête soit sur le statif 1, position active "EXAM", soit sur les rails 32, position d'attente PARK, un voyant intermédiaire entre ces deux positions, orange marquant que la tête basse est en déplacement. Des connecteurs 50, 60 assurent la liaison avec les systèmes de verrouillage respectivement au statif et aux rails.

L'invention n'est pas limitée au mode de réalisation décrit et représenté. En particulier, tout équipement à deux têtes détectrices susceptible de réaliser des analyses soit tomographique avec une tête, soit corps entier, avec deux têtes, au choix du manipulateur, suivant la position affectée à la tête détectrice basse est du domaine de l'invention.

## Revendications

1. Equipement gamma caméra à deux têtes détectrices comportant, d'une part un statif (1) sur lequel est montée une première tête détectrice (10), et, d'autre part une seconde tête détectrice (30) basse, caractérisé en ce que, pour permettre des examens de deux types, type corps entier utilisant simultanément les deux têtes détectrices et type tomographique n'utilisant que la première tête détectrice, l'équipement comporte des rails de roulement (32) sur lesquels la seconde tête détectrice (30) peut être déplacée entre une position active et une position d'attente en bout de rails, cette seconde tête étant, quelle que soit sa position, alimentée en même temps que la première.

2. Equipement selon la revendication 1, caractérisé en ce que les rails de roulement sur lesquels la seconde tête détectrice (30) est déplaçable sont solidaires d'une platine de liaison sur laquelle s'exerce également le poids du statif (1), le statif formant contrepoids à la seconde tête détectrice (30).

3. Equipement selon la revendication 2, caractérisé en ce qu'une platine de base porte à la fois le chemin de roulement du statif et les rails de roulement de la deuxième tête détectrice et forme ainsi la platine de liaison

4. Equipement selon l'une des revendications 2 et 3 caractérisé en ce que, la seconde tête détectrice (30) étant montée en porte à faux sur un support (31) déplaçable sur les rails (32), un système de jambes de force est prévu en bout de rails pour équilibrer la deuxième tête détectrice en position d'attente.

5. Equipement selon la revendication 4, caractérisé en ce que le système de jambe de force comporte deux jambes de force orthogonales à la direction des rails, écartées d'une distance au moins égale à la longueur sur le rail du support (31).

6. Equipement selon la revendication 4, caractérisé en ce que le système de jambes de force comporte deux jambes de force, obliques par rapport à la direction des rails (32) et réunies en une zone commune.

7. Equipement selon l'une des revendications précédentes caractérisé en ce qu'un système de visualisation des positions de la seconde tête détectrice basse est prévu dans l'équipement.

## Patentansprüche

1. Zwei Detektorköpfe aufweisende Gamma-Kameraeinrichtung mit einerseits einem Ständer (1), auf dem ein erster Detektorkopf (10) getragen ist, und andererseits einem zweiten tiefgelegenen Detektorkopf (30), dadurch gekennzeichnet, daß, um zweierlei Arten von Untersuchungen zuzulassen, nämlich der Ganzkörperart unter gleichzeitiger Verwendung beider Detektorköpfe und der Tomographieart unter Verwendung nur des ersten Kopfes, die Einrichtung Rollschienen (32) umfaßt, auf denen der zweite Detektorkopf (30) zwischen einer aktiven Stellung und einer Wartestellung am Ende der Schienen verschoben werden kann, wobei der zweite Kopf in jeglicher Lage gleichzeitig mit dem ersten Kopf gespeist wird.

2. Einrichtung nach Anspruch 1, dadurch gekennzeichnet, daß die Rollschienen, auf denen der zweite Detektorkopf (30) bewegbar ist, mit einer Verbindungsplatte einstückig verbunden sind, auf der das Gewicht des Ständers (1) ebenfalls zur Wirkung kommt, wobei der Ständer ein Gegengewicht für den zweiten Detektorkopf (30) bildet.

3. Einrichtung nach Anspruch 2, dadurch gekennzeichnet, daß eine Basisplatte gleichzeitig die Bewegungsspur des Ständers und die Rollschienen des zweiten Detektorkopfes trägt, so daß sie ebenfalls die Verbindungsplatte bildet.

4. Einrichtung nach Anspruch 1 oder nach Anspruch 2, dadurch gekennzeichnet, daß der zweite Detektorkopf (30) als Brücke an einer Stütze (31) getragen ist, die auf den Schienen (32) bewegbar ist, ein Strebensystem am Ende der Schienen vorgesehen ist, um den zweite Detektorkopf in der Wartestellung in Gleichgewicht zu halten.

5. Einrichtung nach Anspruch 4, dadurch gekennzeichnet, daß das Strebensystem zwei senkrecht zu der Richtung der Schienen angeordnete Schenkel umfaßt, dessen Zwischenabstand mindestens der Länge der Stützschiene (31) entspricht.

6. Einrichtung nach Anspruch 4, dadurch gekennzeichnet, daß das Strebensystem zwei schräg zu der Richtung der Schienen (32) angeordnete und in einem gemeinsamen Bereich miteinander verbundene Schenkel umfaßt.

7. Einrichtung nach einem der vorhergehenden Ansprüche, dadurch gekennzeichnet, daß ein System zur Sichtbarmachung der Stellungen des zweiten tiefgelegenen Detektorkopfes in der Einrichtung vorgesehen ist.

## Claims

1. Gamma camera equipment with two detector heads, comprising a stand on which is mounted a first detector head (10) and also a second detector head (30) lower down, characterized in that, to enable two types of examination to be performed, the complete body type, using the two detector heads simultaneously, and the tomographic type using only the first detector head, the equipment includes running rails (32) over which the second detector head (30) can be moved between an active position and a waiting position at the end of the rails, this second head being fed, whatever its position, at the same time as the first.

2. Equipment according to Claim 1, characterized in that the running rails over which the second detector head (30) is movable are integral with a connecting plate on which the weight of the stand (1) is likewise exerted, the stand forming a counterweight to the second detector head (3).

3. Equipment according to Claim 2, characterized in that a base plate bears both the track of the stand and the running rails of the second detector head, thus forming the connecting plate.

4. Equipment according to either of Claims 2,3, characterized in that the second detector head (30) being mounted in an overhanging position on a support movable over the rails (32), a system of force legs is provided at the end of the rails in order to equilibrate the second detector head in the stand-by position.

5. Equipment according to Claim 4, characterized in that the system of force legs comprises two force legs orthogonal to the direction of the rails and positioned at a distance apart which is at least equal to the length on the supporting rail (31).

6. Equipment according to Claim 4, characterized in that the system of force legs comprises two force legs forming an angle to the direct of the rails (32) and re-combined in a common zone.

7. Equipment according to any one of the preceding claims, characterized in that the system includes a system for the display of the positions of the second detector head mounted lower down.

FIG_1

FIG_2

# FIG_3

# FIG_4